# EUROPEAN PATENT SPECIFICATION

(11) **EP 2 178 501 B1**
(45) Date of publication and mention of the grant of the patent: **02.05.2018**
(21) Application number: 08801496.4
(22) Date of filing: 28.07.2008
(51) Int. Cl.: A61K 9/08, A61L 2/03

(54) **PHARMACEUTICAL PREPARATIONS COMPRISING ELECTROCHEMICALLY ACTIVATED HYPOCHLORITE SOLUTIONS**
PHARMAZEUTISCHE ZUBEREITUNGEN MIT ELEKTROCHEMISCH AKTIVIERTEN HYPOCHLORITLÖSUNGEN
PRÉPARATIONS PHARMACEUTIQUES COMPRENANT DES SOLUTIONS D'HYPOCHLORITE ACTIVÉES ÉLECTROCHIMIQUEMENT

(30) Priority: 26.07.2007 US 952035 P; 15.11.2007 US 988181 P
(43) Date of publication of application: 28.04.2010
(73) Proprietor: Azad Pharma AG, 3125 Toffen (CH)
(72) Inventor: BARONIAN, Mihran, CH-3125 Toffen (CH)
(74) Representative: Weickmann & Weickmann PartmbB
(86) International application number: PCT/EP2008/006193
(87) International publication number: WO 2009/013019

(56) References cited:
- WO-A-00/24431
- WO-A-00/33757
- WO-A-01/56616
- Auf der Basis des nachfolgend beschriebenen Wirkstoffes Annolyte ® bzw. IMECALYTE ® haben wir Produkte für den Veterinärbereich entwickelt und während 2005/06 in Zusammenarbeit mit Veterinären ausführlich getestet: z.b. DECALYTE`D © gegen Ohrenpilze bei Hunden created 13.7.2006 XP002512009

## Description

The present application refers to pharmaceutical preparations comprising an active ingredient and a carrier wherein the carrier comprises an aqueous electrochemically activated salt solution.

Annolyte® or Imecalyte® is a neutral electrochemically activated salt solution, which is a highly effective disinfectant. This activated solution may be obtained by electrolysis of sodium chloride solutions. It can be used in applications like surface disinfection, e.g. of working plates, tables, floors, etc., for cold sterilizing procedures, in agriculture for the elimination of microbial organisms, for wash and laundry applications in swimming pools and even as prophylaxis against athlete's foot. A device for manufacturing neutral electrochemically activated salt solutions is described in EP-A-1 728 768. The Applicant, however, has no knowledge of these solutions as carriers for use in pharmaceutical compositions.

WO 2004/031077 discloses a device for producing a biocidal solution by electrolytic treatment of an aqueous salt solution.

WO 2005/065383 discloses an oxidative reduction potential water solution and its use as disinfectant or for wound treatment.

The package information leaflet on ECALYTE©, DECALYTE©, DECALYTE'D© (XP002512009) describes the veterinary use of these substances.

WO 00/24431 discloses a method for antibacterial treatment of a contaminated medium with electrochemically activated bactericidal aqueous solution by atomizing and dispersing the electrochemically activated bactericidal aqueous solution into the atmosphere to a contaminated medium to be treated.

WO 00/33757 discloses a decontamination system that includes electrolyzed water, which is produced by electrolysis.

WO 01/56616 relates to dental equipment comprising a dental unit having an electrolytic device for producing an electrolytically activated microcidal aqueous solution for use in the elimination and control of biofilm in dental unit waterlines.

Subject-matter of the present invention is the use of aqueous electrochemically activated salt, particularly hypochlorite salt solutions as carriers in pharmaceutical preparations. The pharmaceutical preparationspreferably comprise at least two separate phases, wherein a first phase comprises the active ingredient and a second phase comprises the carrier.

The present invention is defined by the enclosed claims.

In a first aspect the present invention refers to a pharmaceutical preparation comprising an active ingredient and a carrier, wherein the carrier comprises an aqueous electrochemically activated salt solution having a content of free chlorine between 1 and 500 mg/l and a positive redox potential of between +150 and +1350 mV. The active ingredient is preferably physically separated from the carrier, e.g. the active ingredient is present in a phase separate from the electrochemically activated salt solution. If the active ingredient is, however, sufficiently stable in the presence of the electrochemically activated salt, the pharmaceutical preparation may also consist of a single phase, e.g. an aqueous solution.

In a further aspect the present invention refers to the use of an aqueous electrochemically activated salt having a content of free chlorine between 1 and 500 mg/l and a positive redox potential of between +150 and +1350 mV as a carrier for use in human medicine.

The aqueous electrochemically activated solution has a content of free chlorine (as determined by amperometric measurement (DPD) according to US 4,278,507), which provides sufficient activity, e.g. disinfectant or anti-microbial activity, without detrimentally affecting the stability of the preparation. The content of free chlorine may be adjusted by diluting concentrated electrochemically activated salt solutions in a ratio of e.g. 1 part (vol) salt solution to from 1 to 250 parts (vol) of a physiologically acceptable carrier such as water, buffer or a saline solution. Preferably, the content of free chlorine is between 1 and 500 mg/l, particularly between 10 and 400 mg/l and more particularly between 100 and 350 mg/l.

The redox potential of the electrochemically activated salt solution is at least between +150 mV, preferably at least +200 mV, more preferably at least +300 mV, even more preferably at least +400 mV, even more preferably at least +500 mV and upt to +1330 mV, preferably up to +1200 mV. In certain embodiments, the redox potential is +650 and +950 mV, particularly between +700 and +900 mV. The electrochemically activated salt solution is preferably an alkaline metal hypochlorite solution, e.g. lithium, sodium or potassium hypochlorite solution. More preferably, the solution is a sodium hypochlorite solution.

The electrochemically activated salt solution usually has a pH from 2-8. In certain embodiments the pH may be from 2-5, e.g. from 2-4, from 2-3 or from 2-2.8. In other embodiments, the pH may be from 5-8, particularly from 5.9 to 7.6 and more particularly from 6.7 to 7.4.

The content of chlorate and/or the content of chlorite is preferably below toxic levels, e.g. less than 10 mg/l. Further, the solution is preferably free from detectable amounts of radicals such as OH radicals and from ozone. Furthermore, the solution is preferably free from heavy metal ions, e.g. from Mo ions.

The active ingredient can be any medicament suitable for use in human or veterinary medicine, e.g. selected from hydrophilic active ingredients or from lipophilic agents. In certain aspects, it is preferred that the active ingredient is selected from lipophilic or amphiphilic ingredients, i.e. ingredients, which have a butanol-water distribution coefficient of at least 0.5, preferably of at least 1. In other aspects, the active ingredient is a hydrophilic ingredient such as polysaccharide. The active agent is selected from agents for the treatment of glaucoma, e.g. prostaglandines such as Latanoprost, beta-blockers such as Timolol, agents for lowering increased intraocular pressure such as Dorzolamide, agents for the treatment of the dry eye syndrome (ophthalmic lubricants), such as hydroxypropylmethylcellulose (hypromellose) and hyaluronic acid and other pharmaceutically active agents.

The pharmaceutical preparation of the present invention is stabilized against microbial degradation. Thus, the preparation is suitable for multi-use applications. The preparation for multi-use applications may even be devoid of conventional preservatives.

The preparation has a stability against microbial degradation of preferably at least 6 months, more preferably at least 12 months even when stored at room temperature. Preferably, the anti-microbial activity of the electrochemically activated salt solution may be determined by measuring the product c x t of concentration (c) and action time (t) according to a method described by Schleupen, GWF, 1996. Preferably, the value of c x t is 1 mg/l x min or less, more preferably 0.5 mg/l x min or less in order to obtain reduction rates of 10⁶ against microorganisms such as *Pseudomonas aeruginosa* or *Legionella pneumophila.*

The preparation may be for any type of administration, e.g. for local or for systemic administration. For example, the preparation is for ocular, nasal, otic, topical, pulmonal, mucosal, oral or intraperitoneal administration, e.g. for administration by injection. Preferred preparations are for ocular administration, e.g. for the treatment of glaucoma or of the dry eye syndrome.

In one preferred embodiment of the invention, the pharmaceutical preparation comprises a single phase comprising both the active ingredient and the electrochemically activated salt. In this embodiment, the active ingredient is sufficiently stable against degradation in the presence of the chemically activated salt. Herein, the active ingredients are polysaccharides and polyvinylpyrrolidone polymers. The polysaccharides may e.g. be selected from cellulose or cellulose derivatives or glucosamino glycanes such as heparin, heparane sulfate and hyaluronic acid. In one embodiment, the preparation may comprise an ophthalmic lubricant and a carrier, wherein the carrier comprises an aqueous electrochemically activated salt solution having a content of free chlorine as described above, i.e. between 1 and 500 mg/l and a redox potential as described above, i.e. between +150 and +1350 mV. The ophthalmic lubricant may be a polymer, e.g. polysaccharide or a polyvinyl-pyrrolidone, for example cellulose or a cellulose derivative such as hydroxypropylmethylcellulose or a glucosamine glycane such as hyaluronic acid. The pharmaceutical preparation is preferably a homogenous aqueous solution. In this embodiment it is preferred that no further active agents and/or no preservatives are present in the preparation. The preparation according to said embodiment can be used for treating and/or preventing the dry eye syndrome.

In another embodiment, the preparation preferably forms at least two separate phases wherein the active agent is present in a first phase and the electrochemically activated salt solution is present in a second phase separate from the first phase. The first phase may be a solid particulate phase, a liquid hydrophobic phase or a solid or liquid phase having a barrier towards the second phase which is an aqueous phase comprising the carrier. Thus, the preparation may be an emulsion, e.g. a microemulsion, or a liposomal preparation, or a microcapsule preparation, or dispersion wherein the active agent may be emulgated or dispersed optionally in the presence of a carrier, e.g. a lipophilic carrier and/or surfactants, within the aqueous carrier. The active agent may thereby be physically separated from the electrochemically activated salt solution.

Especially preferred are microemulsions as described in EP 07 008 347.1.

In addition to the active agent and the electrochemically activated salt solution, the preparation may contain other known ingredients, e.g. buffers, adjuvants, auxiliary agents, fillers, diluents, etc.

The preparation of the invention may be used in human or veterinary medicine.

Still a further aspect described herein refers to the use of an electrochemically activated salt solution as described above for the cleaning of contact lenses, e.g. glass or plastic contact lenses. In this aspect, the solution may be devoid of any active agent or may comprise an active agent, e.g. a polymer as described above.

Still a further aspect of the present invention refers to the use of an electrochemically activated salt solution as described above for the rinsing of body cavities, e.g. as a solution for the nasal, ocular or otic application. In this embodiment, the solution may be devoid of any active agent, or may comprise an active agent, e.g. a polymer as described above.

Further, the present invention shall be described in more detail by the following examples.

### Example 1

### Preparation and characterisation of microemulsions with an electrochemically activated salt solution as carrier

### 1.1 Composition of tested formulations

The following microemulsions comprising 0.0050% latanoprost as an active ingredient were prepared. All percentages refer to weight percent.

| | ME1 | ME2 | ME3 | ME4 |
|---|---|---|---|---|
| Latanoprost [%] | 0.0050 | 0.0050 | 0.0050 | 0.0050 |
| Ethyl oleate [%] | 4.38 | 4.38 | 4.01 | 3.02 |
| Tween 80 [%] | 4.52 | 4.53 | 4.01 | 3.02 |
| Tween 20 [%] | 2.21 | 2.21 | 2.01 | 1.46 |
| Water [%] | 75.90 | 85.55 | 66.96 | 50.01 |
| Sodium Hyaluronate [%] | 0.025 | 0.025 | 0.020 | 0.020 |
| Sodium Citrate [%] | 0.90 | 0.89 | 0.76 | 0.58 |
| Sorbitol [%] | 1.92 | 1.82 | 1.61 | 1.18 |
| Calcium Citrate [%] | 0.010 | 0.010 | 0.0041 | 0.0032 |
| IMECALYTE® [%] | 10.11 | 0.56 | 20.61 | 40.7 |
| Redox Potential [mV] | 244 | 188 | 325 | 398 |

### 1.2 Stability of latanoprost in IMECALYTE® microemulsions

The stability of latanoprost in microemulsions comprising an electrochemically activated salt solution (IMECALYTE®) as a carrier was determined by HPLC. As comparison, the stability of latanoprost in IMECALYTE® solutions was tested.

The amount of latanoprost (µg/ml) after storage at room temperature for the indicated time periods was as follows:

| **Room temperature** | **ME1 10% IMECALYTE®** | **ME2 0.5% IMECALYTE®** | **Pure solution 0.5% IMECALYTE®** | **Pure solution 10% IMECALYTE®** |
|---|---|---|---|---|
| t=0 | 60.3 | 60.2 | 58.8 | 58.8 |
| t=5 min | | | | 2.1 |
| t= 30 min | | | | n.d. |
| t= 2 weeks | 60.2 | 60.3 | 48.2 | - |
| t=4 weeks | 60.4 | 60.3 | 35.4 | - |
| t=12 weeks | 59.6 | 60.2 | 17.4 | - |

The results show that latanoprost is protected from degradation induced by IMECALYTE® in microemulsion formulations ME1 and ME2. In contrast thereto, latanoprost is unstable in IMECALYTE® solutions. In 0.5% IMECALYTE®, significant degradation is detected after two weeks. In a 10% IMECALYTE® solution, latanoprost is completely degraded within a few minutes.

### 1.3 Antimicrobial activity

The antimicrobial growth activity of latanoprost containing microemulsions ME1-4 against *Staphylococcus aureus* and *Pseudomonas aeruginosa* was tested. The starting concentration of S. *aureus* was 2.82 x 10⁵/ml for ME1 and ME2 and 1.7 x 10⁶/ml for ME3 and ME4. The starting concentration of P. *aeruginosa* was 2.15 x 10⁶/ml for ME1 and ME2, 3.18 x 10⁵/ml for ME3 and 3.18 x 10⁶/ml for ME4.

After 14 days, no viable *S*. *aureus* and *P*. *aeruginosa* microorganisms (concentration <10/ml) could be detected in ME1, ME3 and ME4. In ME2, after 28 days, no S. *aureus* organisms were detectable. Growth of *P. aeruginosa,* however, could not be significantly inhibited in ME1.

The result of Example 1 shows that an active ingredient such as latanoprost is protected from degradation in IMECALYTE®-based microemulsions. Further, these microemulsions exhibit significant antibacterial properties.

### Example 2

### Preparation and characterisation of IMECALYTE®-based hyaluronic acid formulations

The following hyaluronic acid formulations were prepared. All percentages are weight percentages.

| | **HS1** | **HS2** |
|---|---|---|
| Sodium Hyaluronate [%] | 0.30 | 0.30 |
| Sodium citrate [%] | 0.85 | 0.96 |
| Sorbitol [%] | 1.78 | 1.99 |
| Water for inject. [%] | 86.90 | 96.25 |
| IMECALYTE® [%] | 10.17 | 0.50 |
| Redox Potential [mV] | 367 | 181 |

It was tested if IMECALYTE® has a relevant effect on formulation properties such as drop erogation. The contact angle of erogated drops on a reference paper surface was measured immediately after manufacture of formulations HS1 and HS2 and after storage for three months at room temperature. As comparison, a hyaluronic acid formulation corresponding to HS2 (however without IMECALYTE®) was used.

The following results were obtained:

| | **Contact angle t=0** | **Contact angle t=3 months** |
|---|---|---|
| HS2 no IMECALYTE® | 120.7° +/- 6.3 | 121.5° +/- 4.8 |
| HS2 | 118.8° +/- 3.3 | 119.6° +/- 5.2 |
| HS1 | 116..9° +/- 5.6 | 118.4° +/- 5.6 |

Further, the spreading of erogated drops of different hyaluronic acid formulations immediately after manufacture (t=0) and after storage for three months at room temperature (t= 3 months) was determined.

| | **DROP DIAMETER 2R mm** | | |
|---|---|---|---|
| | t=5 sec | t=30 sec | t=300 sec |
| *at t=0* | | | |
| HS2 no IMECAL. | 1.8 | 1.7 | 2.0 |
| HS2 | 1.4 | 1.5 | 1.5 |
| HS1 | 1.5 | 1.4 | 1.4 |
| *at t*=*3 months at RT* | | | |
| HS2 no IMECAL. | 1.9 | 2.0 | 1.9 |
| HS2 | 1.5 | 1.4 | 1.5 |
| HS1 | 1.6 | 1.5 | 1.5 |

The above results show that IMECALYTE® has no relevant effect on the drop erogation characteristics of hyaluronic acid formulations. The quality of erogation and drop formation of the IMECALYTE®-based solutions was very good.

Further, the antimicrobial activity of IMECALYTE®-based hyaluronic acid formulations HS1 and HS2 was determined. As test organisms, *S*. *aureus* (starting concentration 7.27 x 10⁵/ml) and *P. aeruginosa* (starting concentration 1.29 x 10⁶/ml) were used. With solution HS1, the concentration of viable *P*. *aeruginosa* was below 10²/ml after 6h. With *S*. *aureus,* no viable microbes could be determined after 14 days. With the formulation HS2, viable *P. aeruginosa* organisms could not be detected after 7 days. Viable *S*. *aureus* organisms could not be detected after 14 days.

The above results show that IMECALYTE®-based hyaluronic acid formulations are both stable and antimicrobially active.

## Claims

1. Pharmaceutical preparation comprising an active agent, e.g. prostaglandins, beta-blockers, agents for lowering increased intraocular pressure, or ophthalmic lubricants, and a carrier, wherein the carrier comprises an aqueous electrochemically activated salt solution having a content of free chlorine between 1 and 500 mg/l and a redox potential of between +150 and +1350 mV, wherein the active agent is present in a phase separate from the electrochemically activated salt solution.

2. The preparation of claim 1, wherein the content of free chlorine is between 10 and 400 mg/l, particularly between 100 and 350 mg/l and/or the redox potential is between +650 and +950 mV, particularly between +700 and +900 mV.

3. The preparation of any one of claims 1 or 2, wherein the electrochemically activated salt solution is a sodium hypochlorite solution, particularly having a pH from 2 to 8.

4. The preparation of any one of claims 1-3, wherein the content of chlorate and/or the content of chlorite is less than 10 mg/l.

5. The preparation of any one of claims 1-4, which is for multi-use application, in particular for local or for systemic administration, and/or for ocular, nasal, otic, topical, pulmonal, mucosal, oral or intraperitoneal administration.

6. The preparation of any one of claims 1-5, wherein the active agent is present in a solid particulate phase, a liquid hydrophobic phase or a solid or liquid phase having a barrier towards the electrochemically activated salt solution, particularly the preparation is an emulsion or dispersion.

7. Aqueous electrochemically activated salt solution having a content of free chlorine between 1 and 500 mg/l and a redox potential of between +150 and +1350 mV as a carrier for use in human medicine.

8. Aqueous electrochemically activated salt solution for use according to claim 7 for the rinsing of body cavities for nasal, ocular or otic application.

9. Pharmaceutical preparation comprising an ophthalmic lubricant and a carrier wherein the carrier comprises an aqueous electrochemically activated salt solution having a content of free chlorine between 1 and 500 mg/l and a redox potential of between +150 and +1350 mV.

10. The preparation of claim 9, wherein the preparation is an aqueous solution.

11. The preparation of claim 9 or 10, wherein the ophthalmic lubricant is a polysaccharide or polyvinylpyrrolidone polymer such as cellulose, a cellulose derivative, e.g. hydroxypropylcellulose, or a glucosamine glycane, e.g. hyaluronic acid.

12. The preparation of any one of claims 9-11, wherein no further active agents and/or preservatives are present.

13. The preparation of any one of the claims 9 to 12 for use in the treatment and/or prevention of the dry eye syndrome.

14. Pharmaceutical preparation comprising an active ingredient and a carrier, wherein the carrier comprises an aqueous electrochemically activated salt solution having a content of free chlorine between 1 and 500 mg/l and a redox potential of between +150 and +1350 mV, and the active ingredient is a polysaccharide or a polyvinylpyrrolidone polymer such as cellulose, a cellulose derivative, or a glucosamine glycane, e.g. hyaluronic acid.

## Patentansprüche

1. Pharmazeutische Zubereitung umfassend einen aktiven Wirkstoff, z.B. Prostaglandine, Betablocker, Wirkstoffe zur Senkung von erhöhtem intraokulärem Druck oder Augenschmiermittel, und einen Träger, wobei der Träger eine wässrige elektrochemisch aktivierte Salzlösung umfasst, die einen Gehalt an freiem Chlor zwischen 1 und 500 mg/l und ein Redoxpotential von zwischen +150 und +1350 mV aufweist, wobei der aktive Wirkstoff in einer Phase vorliegt, die von der elektrochemisch aktivierten Salzlösung separat ist.

2. Zubereitung nach Anspruch 1, wobei der Gehalt an freiem Chlor zwischen 10 und 400 mg/l liegt, insbesondere zwischen 100 und 350 mg/l und/oder das Redoxpotential zwischen +650 und +950 mV liegt, insbesondere zwischen +700 und +900 mV.

3. Zubereitung nach einem der Ansprüche 1 oder 2, wobei die elektrochemisch aktivierte Salzlösung eine Natriumhypochloritlösung ist, die insbesondere einen pH von 2 bis 8 aufweist.

4. Zubereitung nach einem der Ansprüche 1-3, wobei der Gehalt an Chlorat und/oder der Gehalt an Chlorit weniger als 10 mg/l ist.

5. Zubereitung nach einem der Ansprüche 1-4, die für die mehrmalige Anwendung dient, insbesondere für lokale oder für systemische Verabreichung und/oder für okuläre Verabreichung, nasale Verabreichung, Verabreichung über das Ohr, topische Verabreichung, pulmonale Verabreichung, mukosale Verabreichung, orale Verabreichung oder intraperitoneale Verabreichung.

6. Zubereitung nach einem der Ansprüche 1-5, wobei der aktive Wirkstoff in einer festen partikelförmigen Phase, einer flüssigen hydrophoben Phase oder einer festen oder flüssigen Phase, die eine Barriere gegenüber der elektrochemisch aktivierten Salzlösung hat, vorliegt, insbesondere ist die Zubereitung eine Emulsion oder Dispersion.

7. Wässrige elektrochemisch aktivierte Salzlösung, die einen Gehalt an freiem Chlor zwischen 1 und 500 mg/l und ein Redoxpotential von zwischen +150 und +1350 mV aufweist, als ein Träger zur Verwendung in der Humanmedizin.

8. Wässrige elektrochemisch aktivierte Salzlösung zur Verwendung gemäß Anspruch 7 zum Spülen von Körperhöhlen für die nasale Anwendung, okuläre Anwendung oder die Anwendung über das Ohr.

9. Pharmazeutische Zubereitung umfassend ein Augenschmiermittel und einen Träger, wobei der Träger eine wässrige elektrochemisch aktivierte Salzlösung umfasst, die einen Gehalt an freiem Chlor zwischen 1 und 500 mg/l und ein Redoxpotential von zwischen +150 und +1350 mV aufweist.

10. Zubereitung nach Anspruch 9, wobei die Zubereitung eine wässrige Lösung ist.

11. Zubereitung nach Anspruch 9 oder 10, wobei das Augenschmiermittel ein Polysaccharid- oder Polyvinylpyrrolidon-Polymer wie beispielsweise Cellulose, ein Cellulosederivat, z.B. Hydroxypropylcellulose, oder ein Glucosaminglycan, z.B. Hyaluronsäure, ist.

12. Zubereitung nach einem der Ansprüche 9-11, wobei keine weiteren aktiven Wirkstoffe und/oder Konservierungsstoffe vorliegen.

13. Zubereitung nach einem der Ansprüche 9 bis 12 zur Verwendung bei der Behandlung und/oder Vorbeugung von Trockenes-Auge-Syndrom.

14. Pharmazeutische Zubereitung umfassend eine Wirksubstanz und einen Träger, wobei der Träger eine wässrige elektrochemisch aktivierte Salzlösung umfasst, die einen Gehalt an freiem Chlor zwischen 1 und 500 mg/l und ein Redoxpotential von zwischen +150 und +1350 mV aufweist, und die Wirksubstanz ein Polysaccharid- oder ein Polyvinylpyrrolidon-Polymer wie beispielsweise Cellulose, ein Cellulosederivat oder ein Glucosaminglycan, z.B. Hyaluronsäure, ist.

## Revendications

1. Préparation pharmaceutique comprenant un agent actif, par exemple des prostaglandines, des bêtabloquants, des agents pour baisser une pression intraoculaire augmentée, ou des lubrifiants ophtalmiques, et un vecteur, dans laquelle le vecteur comprend une solution saline activée électrochimiquement aqueuse ayant une teneur en chlore libre entre 1 et 500 mg/L et un potentiel d'oxydoréduction entre + 150 et + 1 350 mV, dans laquelle l'agent actif est présent dans une phase séparée de la solution saline activée électrochimiquement.

2. Préparation selon la revendication 1, dans laquelle la teneur en chlore libre est entre 10 et 400 mg/L, en particulier entre 100 et 350 mg/L et/ou le potentiel d'oxydoréduction est entre + 650 et + 950 mV, en particulier entre + 700 et + 900 mV.

3. Préparation selon l'une quelconque des revendications 1 ou 2, dans laquelle la solution saline activée électrochimiquement est une solution d'hypochlorite de sodium, ayant en particulier un pH de 2 à 8.

4. Préparation selon l'une quelconque des revendications 1 à 3, dans laquelle la teneur en chlorate et/ou la teneur en chlorite sont inférieures à 10 mg/L.

5. Préparation selon l'une quelconque des revendications 1 à 4, qui est destinée à une application à usage multiple, notamment pour une administration locale ou systémique, et/ou pour une administration oculaire, nasale, otique, topique, pulmonaire, par voie muqueuse, orale ou intrapéritonéale.

6. Préparation selon l'une quelconque des revendications 1 à 5, dans laquelle l'agent actif est présent dans une phase particulaire solide, une phase hydrophobe liquide ou une phase solide ou liquide ayant une barrière vis-à-vis de la solution saline activée électrochimiquement, en particulier la préparation est une émulsion ou une dispersion.

7. Solution saline activée électrochimiquement aqueuse ayant une teneur en chlore libre entre 1 et 500 mg/L et un potentiel d'oxydoréduction entre + 150 et + 1 350 mV en tant que vecteur pour utilisation en médecine humaine.

8. Solution saline activée électrochimiquement aqueuse pour utilisation selon la revendication 7 pour rincer des cavités corporelles pour une application nasale, oculaire ou otique.

9. Préparation pharmaceutique comprenant un lubrifiant ophtalmique et un vecteur, dans laquelle le vecteur comprend une solution saline activée électrochimiquement aqueuse ayant une teneur en chlore libre entre 1 et 500 mg/L et un potentiel d'oxydoréduction entre + 150 et + 1 350 mV.

10. Préparation selon la revendication 9, dans laquelle la préparation est une solution aqueuse.

11. Préparation selon la revendication 9 ou 10, dans laquelle le lubrifiant ophtalmique est un polymère de polysaccharide ou poly(vinylpyrrolidone) tel que la cellulose, un dérivé de cellulose, par exemple l'hydroxypropylcellulose, ou un glucosamine glycane, par exemple l'acide hyaluronique.

12. Préparation selon l'une quelconque des revendications 9 à 11, dans laquelle aucun agent actif supplémentaire et/ou conservateur n'est présent.

13. Préparation selon l'une quelconque des revendications 9 à 12 pour utilisation dans le traitement et/ou la prévention du syndrome de l'oeil sec.

14. Préparation pharmaceutique comprenant un ingrédient actif et un vecteur, dans laquelle le vecteur comprend une solution saline activée électrochimiquement aqueuse ayant une teneur en chlore libre entre 1 et 500 mg/L et un potentiel d'oxydoréduction entre + 150 et + 1 350 mV, et l'ingrédient actif est un polymère de polysaccharide ou poly(vinylpyrrolidone) tel que la cellulose, un dérivé de cellulose, ou un glucosamine glycane, par exemple l'acide hyaluronique.
